# FASCICULE DE BREVET EUROPEEN

(11) **EP 3 237 063 B1**
(45) Date de publication et mention de la délivrance du brevet: **20.11.2019**
(21) Numéro de dépôt: 15817255.1
(22) Date de dépôt: 18.12.2015
(51) Int. Cl.: A61N 5/06, A61N 1/36

(54) **DISPOSITIF IMPLANTABLE DE STIMULATION OPTIQUE DU CERVEAU COMPORTANT UN CATHÉTER À CANAUX MULTIPLES**
IMPLANTIERBARE VORRICHTUNG ZUR OPTISCHEN STIMULATION DES GEHIRNS MIT EINEM MEHRKANALIGEN KATHETER
IMPLANTABLE DEVICE FOR OPTICALLY STIMULATING THE BRAIN COMPRISING A MULTI-CHANNEL CATHETER

(30) Priorité: 26.12.2014 FR 1463353
(43) Date de publication de la demande: 01.11.2017
(73) Titulaire: Commissariat à l'Énergie Atomique et aux Énergies Alternatives, 75015 Paris (FR)
(72) Inventeur: CHABROL, Claude, 38320 Poisat (FR); BENABID, Alim-Louis, 38240 Meylan (FR)
(74) Mandataire: Brevalex
(86) Numéro de dépôt international: PCT/EP2015/080444
(87) Numéro de publication internationale: WO 2016/102351

(56) Documents cités:
- EP-A1- 2 641 528
- US-A- 5 125 925
- US-A- 5 728 092
- US-A1- 2002 087 206
- US-A1- 2009 054 955
- US-A1- 2009 118 800
- US-A1- 2012 253 261
- US-A1- 2013 317 573

## Description

### DOMAINE TECHNIQUE

La présente invention se rapporte au domaine de la stimulation profonde du cerveau d'un être humain ou d'un animal, et plus particulièrement au domaine de la stimulation profonde du cerveau par irradiation optique.

La stimulation cérébrale profonde (ou encore « deep brain stimulation » en anglais) est une technique thérapeutique comportant l'implantation d'un dispositif pour stimuler des parties spécifiques du cerveau. On peut ainsi améliorer différents troubles, liés par exemple à la dépression, la maladie d'Alzheimer ou de Parkinson. L'irradiation optique profonde du cerveau mise en œuvre par l'invention peut notamment permettre le traitement de maladies neurodégénératives telles que la maladie de Parkinson.

L'invention propose ainsi un dispositif implantable de stimulation optique, ou encore d'illumination, du cerveau d'un être humain ou animal.

### ÉTAT DE LA TECHNIQUE ANTÉRIEURE

Des solutions ont déjà été proposées dans l'art antérieur pour permettre le traitement de certains dysfonctionnements neuronaux, dont la maladie de Parkinson, par irradiation optique du cerveau avec une source de lumière émettant dans le domaine de l'infrarouge (IR).

La demande de brevet US 2009/0118800 A1 décrit notamment l'utilisation d'un dispositif implantable dans le cerveau permettant d'irradier optiquement des structures biomoléculaires du cerveau, et en particulier des cellules cibles possédant des protéines photosensibles.

De telles solutions peuvent mettre en œuvre l'introduction d'une fibre optique dans le cerveau, par l'intermédiaire de laquelle de la lumière infrarouge est guidée vers le cerveau à partir d'une source de lumière extérieure au cerveau.

Cependant, la mise en place d'une fibre optique dans le cerveau d'un être humain ou animal présente de nombreux inconvénients et difficultés. En effet, l'insertion de la fibre optique dans le cerveau doit pouvoir être réalisée dans des conditions de sécurité optimales pour le patient. Les fibres optiques n'étant généralement pas biocompatibles, il s'avère nécessaire de disposer d'un moyen de protection stérile et étanche. En outre, la profondeur d'implantation de la fibre optique, c'est-à-dire la longueur de la fibre optique entre le crâne et l'extrémité distale de la fibre optique, est un paramètre variable qui dépend du patient à traiter. Or, le raccordement de l'extrémité proximale de la fibre optique à la source de lumière nécessite un montage optique bien précis qui ne peut pas être réalisé sur mesure au moment de l'intervention chirurgicale pour le cas où la longueur d'implantation prévue de la fibre optique ne serait pas adaptée au patient.

Par ailleurs, US 5,125,925 A, US 5,728,092 A et US 2002/0087206 A1 divulguent d'autres exemples de dispositifs implantables pour stimuler optiquement le cerveau.

### EXPOSÉ DE L'INVENTION

L'invention a pour but de remédier au moins partiellement aux besoins mentionnés ci-dessus et aux inconvénients relatifs aux réalisations de l'art antérieur. L'invention est défini dans les revendication annexées.

L'invention vise notamment à proposer un nouveau type de dispositif implantable de stimulation optique profonde pour l'implantation d'un guide de lumière dans le cerveau, en particulier une fibre optique, l'implantation étant réalisée de façon biocompatible en minimisant les risques médicaux liés à l'utilisation du dispositif, notamment les risques de lésions et/ou d'infections du corps d'un individu.

L'invention a ainsi pour objet, selon l'un de ses aspects, un dispositif implantable de stimulation optique du cerveau d'un être humain ou animal, caractérisé en ce qu'il comporte un cathéter à canaux multiples (ou multicanaux) biocompatible, comprenant une pluralité de canaux s'étendant sensiblement parallèlement entre eux par rapport à un axe longitudinal du cathéter à canaux multiples, le cathéter à canaux multiples comportant une extrémité proximale et une extrémité distale, de préférence obturée, et en ce qu'il comporte en outre :
- un guide de lumière, s'étendant à l'intérieur d'un canal du cathéter à canaux multiples, pour la stimulation optique du cerveau, comportant une extrémité proximale pour recevoir la lumière émise par une source de lumière et une extrémité distale pour délivrer cette lumière à l'intérieur du cerveau, le cathéter à canaux multiples servant de gaine d'enveloppement total du guide de lumière,
- un élément fonctionnel, pouvant être une sonde de surveillance, s'étendant à l'intérieur d'un autre canal du cathéter à canaux multiples, pour la mesure de la lumière injectée dans le milieu ambiant au niveau de l'extrémité distale du guide de lumière, et/ou un élément agissant sur la forme dudit cathéter à canaux multiples.

Grâce à l'invention, l'implantation d'un guide de lumière, notamment une fibre optique, dans le cerveau d'un être humain ou animal peut être réalisée de façon simple, localisée et sécurisée à l'aide du dispositif implantable. Le dispositif implantable peut permettre de réaliser une illumination du cerveau humain ou animal, par exemple dans le proche infrarouge, tout en minimisant les risques médicaux lors de l'implantation et en assurant le confort esthétique et physique de l'individu, le guide de lumière formant une barrière à l'égard des liquides physiologiques. Il peut être utilisé pour l'illumination des tissus du cerveau avec différents objectifs selon l'application visée, telle que la neuro-protection, l'opto-génétique, la stimulation, entre autres.

Par « élément agissant sur la forme dudit cathéter à canaux multiples », on entend par exemple un élément rigide, ou raidisseur, apte à être introduit dans ledit autre canal, de façon à induire une rigidification du canal. Il peut également s'agir d'un élément disposé dans ledit autre canal, de façon à permettre audit cathéter à canaux multiples d'être déformé autour d'une position d'équilibre, puis de regagner ladite position d'équilibre, ladite position d'équilibre étant par exemple courbée.

Le dispositif implantable selon l'invention peut en outre comporter l'une ou plusieurs des caractéristiques suivantes prises isolément ou suivant toutes combinaisons techniques possibles.

De façon avantageuse, le cathéter à canaux multiples est souple de sorte qu'il permet, entre autres, d'éviter tout traumatisme au niveau des tissus de l'individu. Le cathéter à canaux multiples peut ainsi avantageusement être plié selon les besoins, en particulier courbé. Il peut par exemple être réalisé en silicone ou en polyuréthane (PU). Il peut être transparent.

Le cathéter à canaux multiples est avantageusement adapté à son implantation dans le troisième ventricule en passant par l'un des ventricules latéraux.

De plus, le cathéter à canaux multiples peut être fin, se présentant notamment sous une forme cylindrique de diamètre compris entre 1 à 2,2 mm. La finesse du cathéter à canaux multiples est en particulier suffisante pour permettre son insertion dans le troisième ventricule ou en contact de toute autre zone du cerveau accessible chirurgicalement à travers une opération de trépanation de quelques millimètres. Par « opération de trépanation », on fait référence à l'opération courante pratiquée par un neurochirurgien lors de l'implantation d'un cathéter ventriculaire, par exemple pour le traitement de l'hydrocéphalie ou la mise en place d'électrodes de stimulation profonde pour le traitement des tremblements dans le cas de la maladie de Parkinson.

Comme indiqué précédemment, le cathéter à canaux multiples forme une gaine d'enveloppement total du guide de lumière. Autrement dit encore, le guide de lumière peut être totalement surmoulé par le cathéter à canaux multiples (enrobage complet), de sorte qu'il soit possible d'éviter toutes fuites de liquides physiologiques, en particulier du sang.

De façon préférentielle, le cathéter à canaux multiples du dispositif implantable selon l'invention peut être préformé pour être conforme à l'anatomie de l'individu, en particulier à l'anatomie des ventricules.

Le guide de lumière est en particulier constitué par un guide de lumière longitudinal souple, notamment une fibre optique.

Par ailleurs, de façon avantageuse, l'élément fonctionnel, notamment la sonde de surveillance (ou « monitoring » en anglais), permet de garantir le bon fonctionnement du dispositif et de contrôler la dose de lumière appliquée à l'individu. La mesure de la lumière injectée dans le milieu ambiant permet de connaître la mesure de la lumière injectée dans les tissus de l'individu.

En outre, l'extrémité distale du cathéter à canaux multiples peut présenter une forme oblongue.

De façon avantageuse, une forme oblongue de l'extrémité distale du cathéter à canaux multiples peut permettre de faciliter la pénétration dans les tissus de l'individu.

L'extrémité distale du cathéter à canaux multiples selon l'invention comporte un élément diffuseur de lumière, l'élément diffuseur de lumière étant notamment situé au niveau de l'extrémité distale du guide de lumière à l'intérieur du canal dans lequel s'étend le guide de lumière.

L'élément diffuseur peut être adapté en longueur à la surface à traiter du cerveau par stimulation optique. L'élément diffuseur peut notamment s'étendre à l'intérieur du cathéter à canaux multiples, et notamment à l'intérieur du canal du cathéter à canaux multiples dans lequel est situé le guide de lumière, sur une longueur comprise entre 2 et 20 mm, par exemple de l'ordre de 10 mm, en fonction de l'application visée. Il est ainsi possible d'obtenir une source linéaire diffusante permettant d'améliorer l'uniformité d'éclairement d'une grande surface du cerveau, notamment du troisième ventricule et de la substance noire compacte du cerveau (SNc ou « Substancia Nigra pars compacta »).

De façon avantageuse, la présence de cet élément diffuseur permet d'éviter l'effet lentille en bout de cathéter à canaux multiples préférentiellement transparent. L'élément diffuseur permet en particulier de limiter les risques de densité de puissance trop élevée. L'élément diffuseur peut notamment comporter une charge de dioxyde de titane (TᵢO₂).

L'élément diffuseur peut permettre de diffuser le rayonnement provenant du guide de lumière, avant qu'il n'atteigne les tissus/cellules du cerveau. On peut ainsi employer une source de lumière générant une densité surfacique de puissance relativement importante, sans risquer l'endommagement des tissus/cellules du cerveau.

De plus, l'élément diffuseur de lumière selon l'invention comporte un fluorophore pour la surveillance par fluorescence.

Un tel fluorophore peut par exemple être constitué par le pigment d'Indocyanine vert (ou encore ICG pour « IndoCyanine Green » en anglais), de longueurs d'ondes d'absorption entre 600 et 900 nm et de longueurs d'onde d'émission entre 750 et 950 nm.

En outre, le cathéter à canaux multiples peut comporter un canal fluidique pour l'injection et/ou le prélèvement de liquides, notamment pour l'injection d'un produit de contraste pendant la phase chirurgicale.

La présence d'un tel canal fluidique peut permettre de faciliter la visualisation des ventricules sans nécessiter la réalisation d'une autre trépanation.

Un tel canal fluidique peut également permettre l'injection de tout autre produit nécessaire au traitement de l'individu. L'extrémité distale d'un tel canal fluidique est avantageusement munie d'un trou ou d'une fente pour permettre l'injection et/ou le prélèvement de liquides.

De plus, le cathéter à canaux multiples peut comporter au moins un canal pour l'utilisation d'un raidisseur pendant la mise en place du cathéter à canaux multiples.

Le cathéter à canaux multiples peut en particulier comporter au moins un canal pour l'utilisation d'un raidisseur localisé en matériau superélastique ou l'utilisation de la capacité d'un guide d'onde thermoplastique à être thermoformé à façon sur le dispositif fini.

Un tel raidisseur peut notamment correspondre à du Nitinol de 250 µm à 500 µm. Il peut se présenter sous la forme d'une tige à mémoire de forme.

En outre, le cathéter à canaux multiples peut comporter un canal équipé d'un marqueur radio-opaque pour le contrôle post-chirurgical.

La présence d'un tel marqueur radio-opaque est avantageusement requise pour faciliter le contrôle du bon positionnement du cathéter à canaux multiples.

Un tel marqueur radio-opaque peut par exemple comporter un liseré d'une charge de baryum sulfate (BaSO₄). Un tel liseré radio-opaque, non transparent, peut être interrompu au niveau du diffuseur éventuel si celui-ci n'est pas directif.

Le cathéter à canaux multiples peut également comporter au moins un canal de passage d'électrodes conductrices pour la stimulation électrique du cerveau au niveau de l'extrémité distale du cathéter à canaux multiples.

Le cathéter à canaux multiples peut aussi comporter un autre canal de passage d'un autre guide de lumière pour la récupération de lumière lors de la surveillance effectuée par le biais de l'élément fonctionnel.

Par ailleurs, le cathéter à canaux multiples peut comporter un moyen de protection d'anti-écrasement et d'anti-pliage, le cathéter à canaux multiples comportant notamment au moins une portion coudée et le moyen de protection d'anti-écrasement et d'anti-pliage étant situé au niveau de ladite au moins une portion coudée.

De façon avantageuse, le moyen de protection d'anti-écrasement et d'anti-pliage permet d'éviter la rupture du guide de lumière pendant l'intervention chirurgicale. Il peut être intégré à l'intérieur du cathéter à canaux multiples ou situé extérieurement à celui-ci et solidarisé par le biais de fixations.

Le guide de lumière peut être réalisé en différents matériaux transparents et d'indice adapté, tels que silice, silicone ou thermoplastique, tel que du polyméthacrylate de méthyle (PMMA). Il peut alors comporter au moins une portion coudée mise en forme par chauffage localisé. Une telle mise en forme peut être réalisée lors de la chirurgie en fonction de l'angle souhaité par le chirurgien grâce à un outillage chauffant localement le guide de lumière au travers du cathéter à canaux multiples, réalisé notamment en silicone. Une température de moulage d'environ 70°C peut être utilisée, très inférieure aux températures de dégradation des silicones, supérieures à 200°C.

Le dispositif implantable selon l'invention peut encore comporter une pluralité d'éléments de connectique au niveau de l'extrémité proximale du cathéter à canaux multiples, et notamment au moins un élément de connectique optique pour la connexion d'au moins un guide de lumière à la source de lumière, un élément de connectique fluidique pour l'injection de produits pendant la phase chirurgicale, notamment d'un produit de contraste, et/ou pour la connexion à une pompe de délivrance implantable pour l'utilisation de produits photosensibles, un élément de connectique électrique pour l'application d'un champ électrique dans la zone illuminée et/ou la réalisation d'une mesure électrique.

En outre, le cathéter à canaux multiples peut comporter un revêtement métallisé, notamment sur la paroi d'au moins un de ses canaux, et notamment le canal comportant le guide de lumière, pour favoriser un angle d'émission de lumière et/ou réaliser une diffusion sélective.

Le dispositif implantable selon l'invention comporte aussi une source de lumière, émettant notamment dans le domaine de l'infrarouge, connectée à au moins l'extrémité proximale du guide de lumière.

La source de lumière peut être intégrée à un neurostimulateur, notamment un neurostimulateur du type à stimulation cérébrale profonde (DBS).

En variante, la source de lumière peut être indépendante d'un neurostimulateur.

Autrement dit encore, la source de lumière peut être déportée d'un neurostimulateur. De façon avantageuse, l'utilisation d'une source de lumière déportée peut permettre d'utiliser des neurostimulateurs commerciaux, voire l'utilisation sur des individus déjà équipés de sondes du type DBS. Cette solution peut en particulier permettre la protection neuronale pour arrêter la dégénérescence neuronale simultanément à une stimulation profonde électrique pour compenser le manque de dopamine chez les individus présentant une phase avancée de la maladie.

La source de lumière peut être située à l'intérieur d'un boîtier hermétique, biocompatible et couplé à au moins un guide de lumière, notamment par le biais d'un connecteur démontable.

Le boîtier peut comporter un détecteur optique pour la surveillance de l'injection de lumière couplé à l'élément fonctionnel et des moyens électroniques de communication sans fil avec un terminal distant pour le contrôle du dispositif.

Par ailleurs, le dispositif implantable selon l'invention peut comporter de plus une source d'alimentation, notamment continue, modulée ou impulsionnelle, par exemple une batterie rechargeable ou une pile, pour alimenter la source de lumière.

En outre, le dispositif implantable selon l'invention peut également être télé-alimenté par le biais d'une antenne d'alimentation externe.

La source de lumière selon l'invention comporte aussi un capteur et un miroir dichroïque, situé entre le capteur et l'élément diffuseur, le capteur permettant la mesure des longueurs d'ondes de retour de l'élément diffuseur émises par fluorescence.

La source de lumière peut notamment émettre dans le domaine du proche infrarouge. La source de lumière peut en particulier être agencée de sorte qu'elle émette une lumière de longueur d'onde préférentiellement comprise entre 650 nm et 950 nm, par exemple de l'ordre de 670 nm.

La source de lumière peut être destinée à être implantée en sous-cutanée, c'est-à-dire sous le cuir chevelu.

La source de lumière peut être constituée par toute source lumineuse capable d'émettre dans l'infrarouge, telle qu'une diode laser, une diode électroluminescente (ou diode LED pour « Light-Emitting Diode » en anglais) ou une diode laser à cavité verticale émettant par la surface (ou diode VCSEL pour « Vertical-Cavity Surface-Emitting Laser en anglais), par exemple. Préférentiellement, la source de lumière comporte une diode laser.

L'invention peut être utiliser dans un procédé de stimulation optique du cerveau d'un être humain ou animal mis en œuvre au moyen d'un dispositif implantable tel que défini précédemment, dans lequel :
(a) on émet une lumière dans le domaine de l'infrarouge, et notamment du proche infrarouge,
(b) on transmet cette lumière à une extrémité proximale d'un guide de lumière implanté dans le cerveau de l'être humain ou animal,
(c) on guide cette lumière dans le guide de lumière jusqu'à une extrémité distale du guide de lumière de sorte que cette lumière irradie l'intérieur du cerveau à partir de son extrémité distale.

Le dispositif tel que défini précédemment pour la stimulation optique du cerveau d'un être humain ou animal peut être implanté par un procédé d'implantation dans lequel on réalise une opération de trépanation pour l'implantation du cathéter à canaux multiples dans le cerveau de l'être humain ou animal.

Le procédé de stimulation optique et/ou le procédé d'implantation peuvent encore comporter au moins l'une des étapes suivantes :
- l'extrémité distale du guide de lumière est implantée à proximité de la substance noire compacte du cerveau (SNc ou « Substancia Nigra pars compacta »),
- l'extrémité distale du guide de lumière est implantée dans le troisième ventricule du cerveau, lequel est situé à proximité de la substance noire compacte,
- l'extrémité distale du guide de lumière est implantée à proximité, voire au contact, du plancher du troisième ventricule du cerveau.

Le procédé de stimulation optique et le procédé d'implantation peuvent comporter l'une quelconque des caractéristiques précédemment énoncées, prises isolément ou selon toutes combinaisons techniquement possibles avec d'autres caractéristiques.

### BRÈVE DESCRIPTION DES DESSINS

L'invention pourra être mieux comprise à la lecture de la description détaillée qui va suivre, d'exemples de mise en œuvre non limitatifs de celle-ci, ainsi qu'à l'examen des figures, schématiques et partielles, du dessin annexé, sur lequel :
- la figure 1 illustre partiellement un exemple de réalisation d'un dispositif implantable conforme à l'invention,
- la figure 2 illustre partiellement, en coupe et en perspective, un exemple de réalisation d'un cathéter à canaux multiples d'un autre dispositif implantable conforme à l'invention, et
- les figures 3 et 4 représentent deux autres exemples de dispositifs implantables conformes à l'invention, comportant respectivement une source de lumière intégrée à un neurostimulateur et une source de lumière déportée par rapport à un neurostimulateur.

Dans l'ensemble de ces figures, des références identiques peuvent désigner des éléments identiques ou analogues.

De plus, les différentes parties représentées sur les figures ne le sont pas nécessairement selon une échelle uniforme, pour rendre les figures plus lisibles.

### EXPOSÉ DÉTAILLÉ DE MODES DE RÉALISATION PARTICULIERS

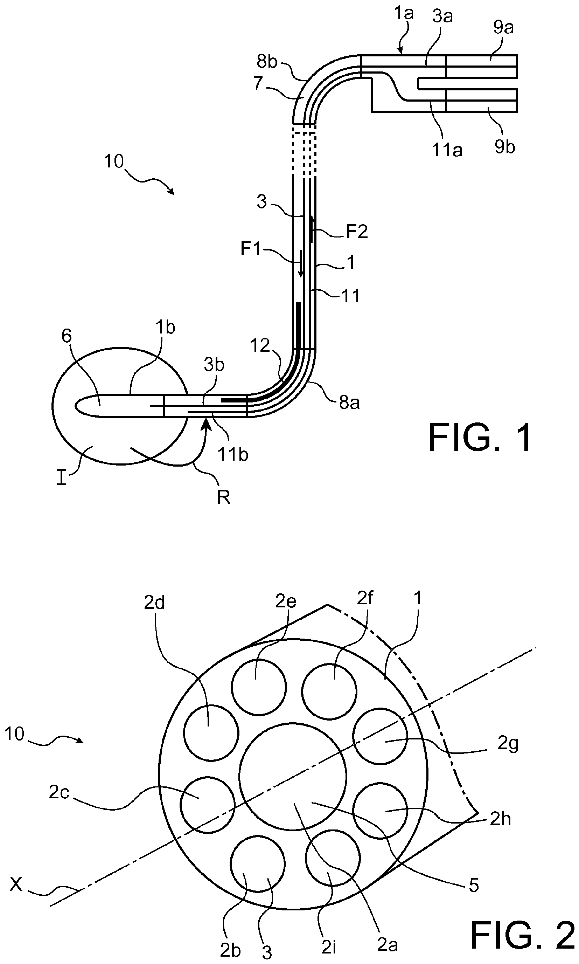

On a illustré sur la figure 1, de façon partielle, un exemple de dispositif implantable 10 conforme à l'invention pour la stimulation optique du cerveau d'un être humain ou animal.

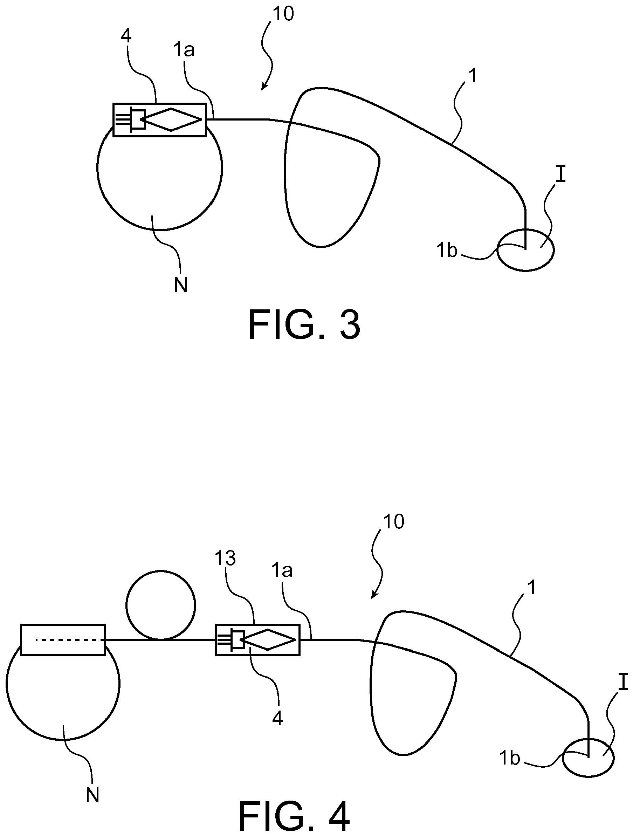

Le dispositif implantable 10 comporte un cathéter à canaux multiples 1 sous la forme d'un tube, pourvu dans cet exemple de deux portions coudées 8a et 8b, d'une extrémité proximale 1a, destinée à être connectée à une source de lumière 4 (visible sur les figures 3 et 4) et éventuellement d'autres systèmes optiques et/ou électroniques, et d'une extrémité distale 1b au niveau de laquelle un guide de lumière 3 émet une illumination I en direction du cerveau humain ou animal.

Le cathéter à canaux multiples 1 est préférentiellement réalisé en silicone ou en polyuréthane (PU), et se présente sous une forme transparente. Il se présente sous une forme cylindrique de diamètre compris entre 1 à 2,2 mm, et sa finesse est telle qu'elle permet son insertion dans le troisième ventricule ou en contact de toute autre zone du cerveau accessible chirurgicalement à travers une opération de trépanation de quelques millimètres.

De plus, afin de faciliter la pénétration dans les tissus, l'extrémité distale 1b du cathéter à canaux multiples 1 peut présenter une forme oblongue, en particulier pour les cathéters de diamètre supérieur à 1,3 mm.

Le cathéter à canaux multiples 1 est avantageusement biocompatible, et comprend une pluralité de canaux 2a-2i (visibles sur la figure 2) qui s'étendent parallèlement entre eux par rapport à l'axe longitudinal X du cathéter 1. Autrement dit encore, sur chaque portion rectiligne du cathéter à canaux multiples 1, les canaux 2a-2i sont parallèles entre eux.

Le guide de lumière 3, notamment sous la forme d'une fibre optique ou guide d'onde fort écart d'indice injecté dans le canal, s'étend dans un premier canal 2b du cathéter 1. Il permet la stimulation optique du cerveau humain ou animal.

Le guide de lumière 3 comporte une extrémité proximale 3a qui reçoit la lumière émise par la source de lumière 4 et une extrémité distale 3b pour délivrer cette lumière à l'intérieur du cerveau sous la forme d'une illumination I.

De façon avantageuse, le cathéter à canaux multiples 1 forme un enrobage total de protection du guide de lumière 3.

Par ailleurs, l'extrémité distale 3b du guide de lumière 3 comporte un élément diffuseur 6 de lumière. Cet élément diffuseur 6 s'étend par exemple à l'intérieur du premier canal 2b du cathéter à canaux multiples 1 dans lequel est situé le guide de lumière 3 sur une longueur de l'ordre de 10 mm. Il est ainsi possible d'obtenir une source linéaire diffusante permettant d'améliorer l'uniformité d'éclairement d'une grande surface du cerveau, et également d'éviter l'effet lentille en bout de cathéter à canaux multiples 1

Cet élément diffuseur 6 comporte en particulier une charge de dioxyde de titane (TᵢO₂). 4. De plus, cet élément diffuseur 6 peut comporter un fluorophore pour la surveillance par fluorescence, constitué par exemple par le pigment d'Indocyanine vert (ou encore ICG pour « IndoCyanine Green » en anglais).

Par ailleurs, sur la figure 1, on constate également que le dispositif implantable 1 peut comporter une pluralité d'éléments de connectique 9a, 9b au niveau de l'extrémité proximale la du cathéter à canaux multiples 1.

En particulier, le cathéter à canaux multiples 1 comporte un élément de connectique optique 9a pour la connexion du guide de lumière 3 à la source de lumière 4.

De plus, le cathéter à canaux multiples 1 comporte également un autre élément de connectique optique 9b qui permet la connexion d'un autre guide de lumière 11, pourvu d'une extrémité proximale 11a et d'une extrémité distale 11b, à la source de lumière 4. Les deux éléments de connectique optique ou embases 9a et 9b peuvent être intégrées si besoin en un seul module de connexion compatible avec un module d'émission/réception optique adapté.

Bien que non représenté, le dispositif implantable 10 peut également comporter un élément de connectique fluidique pour l'injection de produits pendant la phase chirurgicale, notamment d'un produit de contraste, et/ou pour la connexion à une pompe de délivrance implantable pour l'utilisation de produits photosensibles, un élément de connectique électrique pour l'application d'un champ électrique dans la zone illuminée et/ou la réalisation d'une mesure électrique.

Par ailleurs, la première portion coudée 8a peut être rigidifiée par le biais d'une tige à mémoire de forme en matériau super élastique 12, correspondant par exemple à du Nitinol de 250 µm à 500 µm.

En outre, la deuxième portion coudée 8b comporte un moyen de protection d'anti-écrasement et d'anti-pliage 7 de sorte à éviter la rupture des guides de lumière 3 et 11 pendant l'intervention chirurgicale. Ce dispositif anti-écrasement peut par exemple être réalisé en fil spiralé avec comme matériau de l'inox implantable. La longueur de la zone anti-écrasement peut être adaptée en fonction de la longueur implantée dans le cerveau de façon à protéger le coude à 90° à la sortie de la boîte crânienne. Ce moyen de protection 7 peut être intégré à l'intérieur du cathéter à canaux multiples 1 ou situé extérieurement à celui-ci et solidarisé par le biais de fixations.

On a par ailleurs illustré sur la figure 2, en coupe et en perspective, un exemple de réalisation d'un cathéter à canaux multiples 1 d'un autre dispositif implantable 10 conforme à l'invention.

Dans cet exemple, on constate que le cathéter à canaux multiples 1 comporte neuf canaux parallèles entre eux, dont un premier canal 2b dans lequel est situé le guide de lumière 3.

En outre, le cathéter à canaux multiples 1 comporte également un canal central 2a autour duquel sont répartis les huit autres canaux latéraux 2b-2i.

Le canal central 2a comporte alors, conformément à l'invention, une sonde de surveillance 5, qui s'étend à l'intérieur du canal central 2a, pour la mesure de la lumière injectée dans le milieu ambiant au niveau de l'extrémité distale 3b du guide de lumière 3. De façon avantageuse, la sonde de surveillance (ou « monitoring » en anglais) permet de garantir le bon fonctionnement du dispositif 10 et de contrôler la dose de lumière appliquée à l'individu. La mesure de la lumière injectée dans le milieu ambiant permet de connaître la mesure de la lumière injectée dans les tissus de l'individu.

Par ailleurs, comme on peut le voir sur la figure 2, le cathéter à canaux multiples 1 comporte également un canal fluidique 2e pour l'injection et/ou le prélèvement de liquides, et notamment pour l'injection d'un produit de contraste pendant la phase chirurgicale, dont l'extrémité distale est percée pour permettre l'injection et/ou le prélèvement de liquides.

De plus, le cathéter à canaux multiples 1 comporte également deux canaux 2d et 2h pour l'utilisation d'un raidisseur pendant la mise en place du cathéter à canaux multiples 1. Un tel raidisseur est en particulier un raidisseur localisé en matériau superélastique du type du Nitinol à 250 µm.

Afin de faciliter le contrôle du bon positionnement du cathéter à canaux multiples 1, celui-ci comporte également un canal 2f équipé d'un marqueur radio-opaque pour le contrôle post-chirurgical. Ce marqueur radio-opaque peut par exemple comporter un liseré d'une charge de baryum sulfate (BaSO₄).

En outre, le cathéter à canaux multiples 1 comporte deux canaux 2g et 2i de passage d'électrodes conductrices pour la stimulation électrique du cerveau au niveau de l'extrémité distale 1b du cathéter à canaux multiples 1.

Il comporte également un canal 2c de passage d'un autre guide de lumière 11 pour la récupération de lumière lors de la surveillance effectuée par le biais de la sonde de surveillance 5. En effet, comme schématisé sur la figure 1, lorsque le premier guide de lumière 3 envoie de la lumière selon la flèche F1 en direction de l'élément diffuseur 6 pour l'illumination I du cerveau, une partie récupérée R peut être dirigée vers le deuxième guide de lumière 11 qui la renvoie selon la flèche F2 vers la source de lumière 4.

Par ailleurs, comme indiqué précédemment, le dispositif implantable 10 comporte avantageusement une source de lumière 4, émettant dans le domaine de l'infrarouge, connectée aux extrémités proximales 3a et 11a des premier 3 et deuxième 11 guides de lumière.

Les figures 3 et 4 représentent ainsi deux autres exemples de dispositifs implantables 10 conformes à l'invention, comportant respectivement une source de lumière 4 intégrée à un neurostimulateur N et une source de lumière 4 déportée par rapport à un neurostimulateur N.

Dans l'exemple de la figure 3, la source de lumière 4 est intégrée au neurostimulateur N, celui-ci étant notamment du type à stimulation cérébrale profonde (DBS).

En revanche, dans l'exemple de la figure 4, la source de lumière 4 est indépendante du neurostimulateur N, c'est-à-dire qu'elle est déportée du neurostimulateur N.

De façon avantageuse, l'utilisation d'une source de lumière 4 déportée peut permettre d'utiliser des neurostimulateurs commerciaux, voire l'utilisation sur des individus déjà équipés de sondes du type DBS.

Par ailleurs, la source de lumière 4, dans l'exemple de la figure 4, est située à l'intérieur d'un boîtier 13 hermétique, biocompatible et couplé à au moins un guide de lumière 3, notamment par le biais d'un connecteur démontable.

Ce boîtier 13 peut comporter un détecteur optique pour la surveillance de l'injection de lumière couplé à la sonde de surveillance 5 et des moyens électroniques de communication sans fil avec un terminal distant pour le contrôle du dispositif 1.

Le dispositif implantable selon l'invention peut avantageusement permettre de réaliser l'illumination cérébrale profonde sur un être humain ou animal, tout en pouvant être associée à d'autres modes de stimulation, tels que la stimulation électrique ou la stimulation par injection de produit.

Bien entendu, l'invention n'est pas limitée aux exemples de réalisation qui viennent d'être décrits. Diverses modifications peuvent y être apportées par l'homme du métier.

L'expression « comportant un » doit être comprise comme étant synonyme de « comportant au moins un », sauf si le contraire est spécifié.

## Revendications

1. Dispositif implantable (10) de stimulation optique du cerveau d'un être humain ou animal, comportant un cathéter à canaux multiples (1) biocompatible, comprenant une pluralité de canaux (2a-2i) s'étendant sensiblement parallèlement entre eux par rapport à un axe longitudinal (X) du cathéter à canaux multiples (1), le cathéter à canaux multiples (1) comportant une extrémité proximale (1a) et une extrémité distale (1b), le dispositif (10) comportant en outre :
- un guide de lumière (3), s'étendant à l'intérieur d'un canal (2b) du cathéter à canaux multiples (1), pour la stimulation optique du cerveau, comportant une extrémité proximale (3a) pour recevoir la lumière émise par une source de lumière (4) et une extrémité distale (3b) pour délivrer cette lumière à l'intérieur du cerveau, le cathéter à canaux multiples (1) servant de gaine d'enveloppement total du guide de lumière (3),
- un élément fonctionnel (5), s'étendant à l'intérieur d'un autre canal (2a) du cathéter à canaux multiples (1), pour la mesure de la lumière injectée dans le milieu ambiant au niveau de l'extrémité distale (3b) du guide de lumière (3),
l'extrémité distale (1b) du cathéter à canaux multiples (1) comportant un élément diffuseur (6) de lumière comportant un fluorophore pour la surveillance par fluorescence, l'élément diffuseur (6) de lumière étant situé au niveau de l'extrémité distale (3b) du guide de lumière (3) à l'intérieur du canal (2b) dans lequel s'étend le guide de lumière (3), **caractérisé en ce qu'**il comporte en outre une source de lumière (4), émettant dans le domaine de l'infrarouge, connectée à au moins l'extrémité proximale (3a) du guide de lumière (3), et **en ce que** la source de lumière (4) comporte un capteur et un miroir dichroïque, situé entre le capteur et l'élément diffuseur (6), le capteur permettant la mesure des longueurs d'ondes de retour de l'élément diffuseur (6) émises par fluorescence.

2. Dispositif selon la revendication 1, **caractérisé en ce qu'**il comporte en outre un élément agissant sur la forme dudit cathéter à canaux multiples (1).

3. Dispositif selon l'une des revendications précédentes, **caractérisé en ce que** le cathéter à canaux multiples (1) comporte un canal fluidique (2e) pour l'injection et/ou le prélèvement de liquides, notamment pour l'injection d'un produit de contraste pendant la phase chirurgicale.

4. Dispositif selon l'une quelconque des revendications précédentes, **caractérisé en ce que** le cathéter à canaux multiples (1) comporte au moins un canal (2d, 2h) pour l'utilisation d'un raidisseur pendant la mise en place du cathéter à canaux multiples (1).

5. Dispositif selon l'une quelconque des revendications précédentes, **caractérisé en ce que** le cathéter à canaux multiples (1) comporte au moins un canal (2d, 2h) pour l'utilisation d'un raidisseur localisé en matériau superélastique ou l'utilisation de la capacité d'un guide d'onde thermoplastique à être thermoformé à façon sur le dispositif fini.

6. Dispositif selon l'une quelconque des revendications précédentes, **caractérisé en ce que** le cathéter à canaux multiples (1) comporte un canal (2f) équipé d'un marqueur radio-opaque pour le contrôle post-chirurgical.

7. Dispositif selon l'une quelconque des revendications précédentes, **caractérisé en ce que** le cathéter à canaux multiples (1) comporte au moins un canal (2g, 2i) de passage d'électrodes conductrices pour la stimulation électrique du cerveau au niveau de l'extrémité distale (1b) du cathéter à canaux multiples (1).

8. Dispositif selon l'une quelconque des revendications précédentes, **caractérisé en ce que** le cathéter à canaux multiples (1) comporte un autre canal (2c) de passage d'un autre guide de lumière (11) pour la récupération (R) de lumière lors de la surveillance effectuée par le biais de l'élément fonctionnel (5).

9. Dispositif selon l'une quelconque des revendications précédentes, **caractérisé en ce que** le cathéter à canaux multiples (1) comporte un moyen de protection d'anti-écrasement et d'anti-pliage (7), le cathéter à canaux multiples (1) comportant notamment au moins une portion coudée (8b) et le moyen de protection d'anti-écrasement et d'anti-pliage (7) étant situé au niveau de ladite au moins une portion coudée (8b).

10. Dispositif selon l'une quelconque des revendications précédentes, **caractérisé en ce qu'**il comporte une pluralité d'éléments de connectique (9a, 9b) au niveau de l'extrémité proximale (1a) du cathéter à canaux multiples (1), et notamment au moins un élément de connectique optique (9a, 9b) pour la connexion d'au moins un guide de lumière (3, 11) à la source de lumière (4), un élément de connectique fluidique pour l'injection de produits pendant la phase chirurgicale, notamment d'un produit de contraste, et/ou pour la connexion à une pompe de délivrance implantable pour l'utilisation de produits photosensibles, un élément de connectique électrique pour l'application d'un champ électrique dans la zone illuminée et/ou la réalisation d'une mesure électrique.

11. Dispositif selon l'une quelconque des revendications précédentes, **caractérisé en ce que** le cathéter à canaux multiples (1) comporte un revêtement métallisé, notamment sur la paroi d'au moins un de ses canaux (2a-2i), et notamment le canal (2b) comportant le guide de lumière (3), pour favoriser un angle d'émission de lumière et/ou réaliser une diffusion sélective.

12. Dispositif selon l'une quelconque des revendications précédentes, **caractérisé en ce que** la source de lumière (4) est intégrée à un neurostimulateur, notamment un neurostimulateur du type à stimulation cérébrale profonde (DBS), ou est indépendante d'un neurostimulateur.

13. Dispositif selon l'une quelconque des revendications précédentes, **caractérisé en ce que**, la source de lumière (4) étant indépendante d'un neurostimulateur, la source de lumière (4) est située à l'intérieur d'un boîtier (13) hermétique, biocompatible et couplé à au moins un guide de lumière (3), notamment par le biais d'un connecteur démontable, et **en ce que**, optionnellement, le boîtier (13) comporte un détecteur optique pour la surveillance de l'injection de lumière couplé à l'élément fonctionnel (5) et des moyens électroniques de communication sans fil avec un terminal distant pour le contrôle du dispositif (1).

14. Dispositif selon l'une quelconque des revendications précédentes, **caractérisé en ce qu'**il comporte de plus une source d'alimentation, notamment une batterie rechargeable ou une pile, pour alimenter la source de lumière (4).

## Patentansprüche

1. Implantierbare Vorrichtung (10) zur optischen Stimulation des Gehirns eines Menschen oder eines Tiers, umfassend einen biokompatiblen Katheter mit mehreren Kanälen (1), umfassend eine Mehrzahl von Kanälen (2a-2i), die sich im Wesentlichen parallel zueinander bezüglich einer Längsachse (X) des Katheters mit mehreren Kanälen (1) erstrecken, wobei der Katheter mit mehreren Kanälen (1) ein proximales Ende (1a) und ein distales Ende (1b) umfasst, wobei die Vorrichtung (10) ferner umfasst:
- einen Lichtleiter (3), der sich im Inneren eines Kanals (2b) des Katheters mit mehreren Kanälen (1) erstreckt, für die optische Stimulation des Gehirns, umfassend ein proximales Ende (3a) zum Empfangen des Lichts, das von einer Lichtquelle (4) emittiert wird, und ein distales Ende (3b) zum Liefern dieses Lichts ins Innere des Gehirns, wobei der Katheter mit mehreren Kanälen (1) als Ummantelung zur vollständigen Einhüllung des Lichtleiters (3) dient,
- ein funktionales Element (5), das sich im Inneren eines anderen Kanals (2a) des Katheters mit mehreren Kanälen (1) erstreckt, für die Messung des Lichts, das in das Umgebungsmilieu im Bereich des distalen Endes (3b) des Lichtleiters (3) injiziert wird,
wobei das distale Ende (1b) des Katheters mit mehreren Kanälen (1) ein Lichtdiffusorelement (6) umfasst, umfassend ein Fluorophor, für die Überwachung mittels Fluoreszenz, wobei das Lichtdiffusorelement (6) im Bereich des distalen Endes (3b) des Lichtleiters (3) im Inneren des Kanals (2b) angeordnet ist, in dem sich der Lichtleiter (3) erstreckt,
**dadurch gekennzeichnet, dass** sie ferner eine Lichtquelle (4) umfasst, die im Infrarotbereich emittiert, die wenigstens mit dem proximalen Ende (3a) des Lichtleiter (3) verbunden ist, und dass die Lichtquelle (4) einen Sensor und einen dichroitischen Spiegel umfasst, der zwischen dem Sensor und dem Diffusorelement (6) angeordnet ist, wobei der Sensor die Messung der mittels Fluoreszenz emittierten Rückkehrwellenlängen des Diffusorelements (6) erlaubt.

2. Vorrichtung nach Anspruch 1, **dadurch gekennzeichnet, dass** sie ferner ein Element umfasst, das auf die Form des Katheters mit mehreren Kanälen (1) einwirkt.

3. Vorrichtung nach einem der vorhergehenden Ansprüche, **dadurch gekennzeichnet, dass** der Katheter mit mehreren Kanälen (1) einen fluidischen Kanal (2e) für die Injektion und/oder die Entnahme von Flüssigkeiten umfasst, insbesondere für die Injektion eines Kontrastmittels während der chirurgischen Phase.

4. Vorrichtung nach einem der vorhergehenden Ansprüche, **dadurch gekennzeichnet, dass** der Katheter mit mehreren Kanälen (1) wenigstens einen Kanal (2d, 2h) für die Verwendung einer Versteifung während der Platzierung des Katheters mit mehreren Kanälen (1) umfasst.

5. Vorrichtung nach einem der vorhergehenden Ansprüche, **dadurch gekennzeichnet, dass** der Katheter mit mehreren Kanälen (1) wenigstens einen Kanal (2d, 2h) für die Verwendung einer lokalisierten Versteifung aus superelastischem Material oder die Verwendung der Kapazität eines thermoplastischen Wellenleiters zur Thermoformung nach Maß auf der fertiggestellten Vorrichtung umfasst.

6. Vorrichtung nach einem der vorhergehenden Ansprüche, **dadurch gekennzeichnet, dass** der Katheter mit mehreren Kanälen (1) einen Kanal (2f) umfasst, der mit einem radioopaken Marker für die postchirurgische Untersuchtung ausgestattet ist.

7. Vorrichtung nach einem der vorhergehenden Ansprüche, **dadurch gekennzeichnet, dass** der Katheter mit mehreren Kanälen (1) wenigstens einen Kanal (2g, 2i) zur Durchführung von leitenden Elektroden für die elektrische Stimulation des Gehirns im Gereich des distalen Endes (1b) des Katheters mit mehreren Kanälen (1) umfasst.

8. Vorrichtung nach einem der vorhergehenden Ansprüche, **dadurch gekennzeichnet, dass** der Katheter mit mehreren Kanälen (1) einen weiteren Kanal (2c) zur Durchführung eines anderen Wellenleiters (11) für das Auffangen (R) von Licht während der Überwachung umfasst, die mit Hilfe des funktionalen Elements (5) durchgeführt wird.

9. Vorrichtung nach einem der vorhergehenden Ansprüche, **dadurch gekennzeichnet, dass** der Katheter mit mehreren Kanälen (1) ein Anti-Quetsch- und Anti-Knick-Schutzmittel (7) umfasst, wobei der Katheter mit mehreren Kanälen (1) insbesondere einen gebogenen Bereich (8b) umfasst, und wobei das Anti-Quetsch-und Anti-Knick-Schutzmittel (7) im Bereich des wenigstens einen gebogenen Bereichs (8b) angeordnet ist.

10. Vorrichtung nach einem der vorhergehenden Ansprüche, **dadurch gekennzeichnet, dass** sie eine Mehrzahl von Verbindungselementen (9a, 9b) im Bereich des proximalen Endes (1a) des Katheters mit mehreren Kanälen (1) umfasst, und insbesondere wenigstens ein optisches Verbindungselement (9a, 9b) für die Verbindung wenigstens eines Wellenleiters (3, 11) mit der Lichtquelle (4), ein fluidisches Verbindungselement für die Injektion von Mitteln während der chirurgischen Phase, insbesondere eines Kontrastmittels, und/oder für die Verbindung mit einer implantierbaren Förderpumpe für die Verwendung von photosensiblen Mitteln, ein elektrisches Verbindungselement zum Anlegen eines elektrischen Felds in der beleuchteten Zone und/oder die Durchführung einer elektrischen Messung.

11. Vorrichtung nach einem der vorhergehenden Ansprüche, **dadurch gekennzeichnet, dass** der Katheter mit mehreren Kanälen (1) eine metallisierte Beschichtung umfasst, insbesondere auf der Wand von wenigstens einem seiner Kanäle (2a-2i), und insbesondere dem Kanal (2b), der den Wellenleiter (3) umfasst, um einen Emissionswinkel des Lichts zu begünstigen und/oder eine selektive Diffusion zu bewirken.

12. Vorrichtung nach einem der vorhergehenden Ansprüche, **dadurch gekennzeichnet, dass** die Lichtquelle (4) in einen Neurostimulator integriert ist, insbesondere einen Neurostimulator von Typ mit tiefer zerebraler Stimulation (DBS), oder unabhängig von einem Neurostimulator ist.

13. Vorrichtung nach einem der vorhergehenden Ansprüche, **dadurch gekennzeichnet, dass** die Lichtquelle (4) unabhängig von einem Neurostimulator ist, dass die Lichtquelle (4) im Inneren eines hermetischen biokompatiblen Gehäuses (13) angeordnet und mit wenigstens einem Lichtleiter (3) insbesondere mittels eines demontierbaren Verbinders gekoppelt ist, und dass optional das Gehäuse (13) einen optischen Detektor umfasst für die Überwachung der Injektion von Licht, gekoppelt mit dem funktionalen Element (5), sowie drahtlose elektronische Kommunikationsmittel mit einem entfernten Endgerät für die Steuerung der Vorrichtung (1).

14. Vorrichtung nach einem der vorhergehenden Ansprüche, **dadurch gekennzeichnet, dass** sie ferner eine Versorgungsquelle umfasst, insbesondere eine wiederaufladbare Batterie oder eine Primärzelle, um die Lichtquelle (4) zu versorgen.

## Claims

1. An implantable device (10) for optically stimulating the brain of a human being or animal, including a biocompatible multi-channel catheter (1), comprising a plurality of channels (2a-2i) extending substantially in parallel to each other relative to a longitudinal axis (X) of the multi-channel catheter (1), the multi-channel catheter (1) including a proximal end (1a) and a distal end (1b), and further including:
- a light guide (3), extending into a channel (2b) of the multi-channel catheter (1), for optically stimulating the brain, including a proximal end (3a) for receiving light emitted by a light source (4) and a distal end (3b) for delivering this light inside the brain, the multi-channel catheter (1) acting as a sheath for totally wrapping the light guide (3),
- a functional element (5), extending into another channel (2a) of the multi-channel catheter (1), for measuring the light injected into the surrounding medium at the distal end (3b) of the light guide (3),
the distal end (1b) of the multi-channel catheter (1) including a light scattering element (6) including a fluorophore for fluorescence monitoring,
that the light scattering element (6) be located at the distal end (3b) of the light guide (3) inside the channel (2b) into which the light guide (3) extends,
**characterized in that** it further includes a light source (4), emitting in the infrared range, connected to at least the proximal end (3a) of the light guide (3), and **in that** the light source (4) includes a sensor and a dichroic mirror, located between the sensor and the scattering element (6), the sensor enabling wavelengths back from the scattering element (6) emitted by fluorescence to be measured.

2. The device according to claim 1, **characterised in that** it further includes an element acting on the form of said multi-channel catheter (1).

3. The device according to any of the preceding claims, **characterised in that** the multi-channel catheter (1) includes a fluid channel (2e) for injecting and/or sampling liquids, in particular for injecting a contrast agent during the surgical phase.

4. The device according to any of the preceding claims, **characterised in that** the multi-channel catheter (1) includes at least one channel (2d, 2h) for using a stiffener during the placement of the multi-channel catheter (1).

5. The device according to any of the preceding claims, **characterised in that** the multi-channel catheter (1) includes at least one channel (2d, 2h) for using a localised stiffener of super elastic material or using the ability of a thermoplastic waveguide to be custom thermoformed on the finished device.

6. The device according to any of the preceding claims, **characterised in that** the multi-channel catheter (1) includes a channel (2f) equipped with a radiopaque label for post-surgical check.

7. The device according to any of the preceding claims, **characterised in that** the multi-channel catheter (1) includes at least one channel (2g, 2i) for passing conductive electrodes for electrically stimulating the brain at the distal end (1b) of the multi-channel catheter (1).

8. The device according to any of the preceding claims, **characterised in that** the multi-channel catheter (1) includes another channel (2c) for passing another light guide (11) for recovering (R) light during monitoring performed via the functional element (5).

9. The device according to any of the preceding claims, **characterised in that** the multi-channel catheter (1) includes an anti-crushing anti-folding protective means (7), the multi-channel catheter (1) including in particular at least one bent portion (8b) and the anti-crushing anti-folding protective means (7) being located at said at least one bent portion (8b).

10. The device according to any of the preceding claims, **characterised in that** it includes a plurality of connecting elements (9a, 9b) at the proximal end (1a) of the multi-channel catheter (1), and in particular at least one optical connecting element (9a, 9b) for connecting at least one light guide (3, 11) to the light source (4), a fluidic connecting element for injecting products during the surgical phase, in particular a contrast agent and/or for connecting to an implantable delivery pump for using photosensitive products, an electrical connecting element for applying an electric field in the illuminated zone and/or performing an electrical measurement.

11. The device according to any of the preceding claims, **characterised in that** the multi-channel catheter (1) includes a metal coating, in particular on the wall of at least one of its channels (2a-2i), and in particular the channel (2b) including the light guide (3), for promoting a light emitting angle and/or performing a selective scattering.

12. The device according to any preceding claim, **characterised in that** the light source (4) is integrated to a neurostimulator, in particular a deep brain stimulation (DBS) type neurostimulator.

13. The device according to any preceding claim, **characterised in that**, the light source (4) being independent of a neurostimulator, the light source (4) is located inside a sealed biocompatible casing (13) coupled to at least one light guide (3), in particular via a removable connector, and **in that**, optionally, the casing (13) includes an optical detector for monitoring light injection coupled to the functional element (5) and electronic wireless communication means with a remote terminal for checking the device (1).

14. The device according to any preceding claims, **characterised in that** it further includes a power source, in particular a storage battery or a battery cell, for powering the light source (4).
